# EUROPÄISCHE PATENTANMELDUNG

(11) **EP 3 305 332 A1**
(43) Veröffentlichungstag der Anmeldung: **11.04.2018**
(21) Anmeldenummer: 17194015.8
(22) Anmeldetag: 29.09.2017
(51) Int. Cl.: A61L 2/28, G01N 25/18, G01N 27/06, G01N 27/416, G01N 9/00, G01N 21/49

(54) **VERFAHREN ZUM ÜBERPRÜFEN ZUMINDEST EINES DESINFEKTIONSMITTELS**

(30) Priorität: 07.10.2016 DE 102016119096
(71) Anmelder: Brandes Innovation Inh. Ronald Brandes, 26419 Schortens (DE)
(72) Erfinder: Willms, Joachim, 26133 Oldenburg (DE); Brandes, Ronald, 26419 Schortens (DE)
(74) Vertreter: Jabbusch, Matthias

(57) **Zusammenfassung**

Bei einem Verfahren zum Überprüfen zumindest eines Desinfektionsmittels auf seine Desinfektions-Funktion ist vorgesehen, dass zumindest eine Probe des Desinfektionsmittels mit einem Sensor in Bezug auf Änderung mindestens einer physikalischen und/oder chemischen Eigenschaft des Desinfektionsmittels gemessen wird.

## Beschreibung

Die Erfindung betrifft ein Verfahren zum Überprüfen zumindest eines Desinfektionsmittels auf seine Desinfektions-Funktion.

Desinfektionsmittel werden zum Abtöten von Keimen in vielfältigen Umgebungen angewandt. Ein regelmäßiger Einsatz ist in Krankenhäusern, Kinderheimen oder auch Altenheimen gegeben, in denen vermehrt Krankheitserreger auftreten können. Desinfektionsmittel werden beispielsweise in Behältern vorgehalten, aus denen heraus Desinfektionsmittel zum Besprühen der Hände, zum Besprühen von Tüchern oder ähnlich entnommen werden können.

Problematisch ist, dass es in derartigen Krankenhäusern und Heimen häufig zu Drucksituationen hinsichtlich hoher Patienten- oder Heimbewohnerzahlen, geringem Personal, einer großen Anzahl von Praktikanten oder Zivildienstleistenden kommt. Dies alles kann dazu führen, dass ein Desinfektionsmittel in einem konkreten Behälter wochen- oder auch monatelang enthalten ist, ohne dass auf seine Ablaufzeit geachtet wird. Ein derartiges Desinfektionsmittel kann seine Desinfektions-Funktion verlieren, vielmehr kann es sogar mit Keimen belastet sein. Dann würde ein Desinfektionsmittel eine Keimquelle ausbilden.

Der Erfindung liegt die Aufgabe zugrunde, ein Verfahren der eingangs genannten Gattung aufzuzeigen, mit dem ein Desinfektionsmittel zuverlässig auf seine Desinfektions-Funktion überprüfbar ist.

Diese Aufgabe ist erfindungsgemäß dadurch gelöst, dass zumindest eine Probe des Desinfektionsmittels mit einem Sensor in Bezug auf Änderung mindestens einer physikalischen und/oder chemischen Eigenschaft des Desinfektionsmittels gemessen wird.

Das erfindungsgemäße Verfahren schlägt eine aktive Überprüfung von Desinfektionsmitteln während ihrer Verwendung in zum Beispiel Krankenhäusern vor. Nach dem Verfahren wird dem Desinfektionsmittel zumindest eine Probe entnommen, alternativ kann direkt im konkret vorhandenen Desinfektionsmittelvorrat das Verfahren durchgeführt werden. Die gewonnene Probe beziehungsweise das Desinfektionsmittel wird mit einem Sensor vermessen. Dieser kann in automatisierter Weise eine Veränderung einer physikalischen und/oder einer chemischen Eigenschaft des Desinfektionsmittels messen und somit ein Messergebnis feststellen und über geeignete Schnittstellen beziehungsweise Übertragungswege an eine Auswertung liefern. Der Sensor misst die physikalische und/oder chemische Eigenschaft des Desinfektionsmittels. Hat das Desinfektionsmittel noch eine hohe desinfizierende Wirksamkeit, sind seine physikalischen und/oder chemischen Eigenschaften nach den Vorgaben des Herstellers des Desinfektionsmittels. Ist dagegen die Desinfektionseigenschaft des Desinfektionsmittels durch eine höhere Konzentration von Bakterien im Desinfektionsmittel gebunden, ändern sich physikalische und/oder chemische Eigenschaften, was mit dem Sensor festgestellt wird.

Durch eine lange Nutzdauer eines Desinfektionsmittels wird beispielsweise die Leitfähigkeit des Desinfektionsmittels verändert. Diese Änderung kann mit einem Leitfähigkeitssensor festgestellt werden. Alternativ kann der Sensor auch als Lichtsensor ausgebildet sein, wenn nämlich die lange Einsatzzeit eines Desinfektionsmittels zu einer Veränderung seiner Trübung führen.

Weiter alternativ kann der Sensor als pH-Wert-Messer oder als Dichtesensor ausgebildet sein.

## Patentansprüche

1. Verfahren zum Überprüfen zumindest eines Desinfektionsmittels auf seine Desinfektions-Fun ktion,
**dadurch gekennzeichnet,**
**dass** zumindest eine Probe des Desinfektionsmittels mit einem Sensor in Bezug auf Änderung mindestens einer physikalischen und/oder chemischen Eigenschaft des Desinfektionsmittels gemessen wird.

2. Verfahren nach Anspruch 1, **dadurch gekennzeichnet, dass** als Sensor ein Leitfähigkeitssensor eingesetzt wird.

3. Verfahren nach Anspruch 1, **dadurch gekennzeichnet, dass** als Sensor ein Lichtsensor eingesetzt wird.

4. Verfahren nach Anspruch 1, **dadurch gekennzeichnet, dass** als Sensor ein pH-Wert-Messer eingesetzt wird.

5. Verfahren nach Anspruch 1, **dadurch gekennzeichnet, dass** als Sensor ein Dichtesensor eingesetzt wird.
